# EUROPEAN PATENT APPLICATION

(11) **EP 4 012 040 A1**
(43) Date of publication of application: **15.06.2022**
(21) Application number: 20213392.2
(22) Date of filing: 11.12.2020
(51) Int. Cl.: C12P 19/14, C12P 7/10, C08H 8/00

(54) **METHOD FOR CONTROLLING THE PRETREATMENT AND/OR ENZYMATIC HYDROLYSIS OF A LIGNOCELLULOSIC MATERIAL**

(71) Applicant: Sekab E-Technology AB, 891 26 Örnsköldsvik (SE)
(72) Inventor: CAVKA, Adnan, 891 96 Arnäsvall (SE); SUNDVALL, Elias, 892 32 Domsjö (SE)
(74) Representative: Kransell & Wennborg KB

(57) **Abstract**

The present disclosure generally relates to a method and a system for controlling the pretreatment and/or enzymatic hydrolysis of a lignocellulosic material. The method and system allow for savings with respect to energy consumption and costs.

## Description

### Technical field

The present disclosure generally relates to a method and a system for controlling the pretreatment and/or enzymatic hydrolysis of a lignocellulosic material.

### Background

Lignocellulosic residues from forestry are attractive as feedstocks for the production of renewable chemicals and fuels, since they are abundant, relatively inexpensive, and are not used for food. Lignocellulose consists mainly of lignin and two classes of polysaccharides, cellulose and hemicellulose. The polysaccharides can be hydrolyzed to sugars and thereafter converted to various fermentation products, e.g. bio-alcohols, by means of microorganisms, such as baker's yeast *Saccharomyces cerevisiae.*

During enzymatic hydrolysis of lignocellulosic materials, the cellulose present is partly converted into fermentable sugars by cellulolytic enzymes. The hydrolysis of cellulose is typically preceded by a pretreatment process, in which the hemicellulose is degraded, and the cellulose is made increasingly accessible to the enzymes. Enzyme costs typically accounts for a significant proportion of the total costs associated with processes for pretreatment and enzymatic hydrolysis.

A number of pretreatment processes exist, and these may be categorized into the general categories biological, chemical, mechanical and thermal pretreatment processes. Pretreatment methods may also be combined to improve the ultimate cellulose-to-sugar conversion yield.

The general purpose of these pretreatment methods is to change the structure of the lignocellulose such that the enzymatic availability of hydrolysable polymers within the lignocellulosic material is increased. Typically, a change in structure involves a size reduction of the lignocellulosic biomass. Regardless of the pretreatment method used, the change of structure, and particularly the reduction in size of the lignocellulosic biomass is often associated with demanding process conditions and high costs. In particular, if a physical pretreatment step is involved, such as mechanical refining, the process also requires high amounts of energy.

In addition, it is generally difficult to find a streamlined optimal pretreatment process, since the process conditions vary significantly between different lignocellulosic starting materials. This is mainly due to the differences in composition and structural characteristics of the lignocellulosic biomass. For example, the pretreatment process requirements for softwood are different from those for hardwood, and variations within these general groups also exist. The disintegration of spruce may for example often require higher amounts of energy and more harsh pretreatment conditions than e.g. pine. Furthermore, the chemical or physical nature of the ingoing lignocellulosic material will also affect the disintegration, and thereby the overall efficiency of the pretreatment process.

There is therefore a need for a more controlled and cost-efficient pretreatment and hydrolysis process that allows for situation specific adjustments.

### Summary

In view of the above, it is a general object of the present disclosure to provide for improvements with respect to controlling the pretreatment and/or enzymatic hydrolysis. A specific object is to achieve a more controlled and cost-efficient pretreatment and hydrolysis process. Another object is to achieve an energy-efficient pretreatment process of the type involving mechanical refining.

These objects are achieved in accordance with the independent claims.

According to a first aspect of the present disclosure, there is provided a method for controlling the pretreatment and/or enzymatic hydrolysis of a lignocellulosic material comprising:
a) pretreating a lignocellulosic material to form a first slurry comprising lignocellulosic particles,
b) analyzing the lignocellulosic particles of the first slurry to obtain a first data set reflecting the degree of the pretreatment,
c) subjecting the first slurry to mechanical refining to form a second slurry comprising lignocellulosic particles, wherein the average size of the lignocellulosic particles of the second slurry is smaller than the average size of the lignocellulosic particles of the first slurry,
d) controlling the mechanical refining in response to the first data set; and
e) subjecting the second slurry to enzymatic hydrolysis.

The method of the present disclosure provides for improvements with respect to the overall efficiency of the pretreatment and enzymatic hydrolysis and achieves a more controlled and sophisticated pretreatment of lignocellulosic biomass.

The mechanical refining step serves to reduce the size of the lignocellulosic particles and thereby increase the available surface area of the material. This way, the enzymatic availability during the subsequent hydrolysis step is improved.

The present disclosure is based on the realization that by using a mechanical refining step after the primary pretreatment and incorporating an analysis and control mechanism after the pretreatment, the mechanical refining can be more adequately performed. Accordingly, a more efficient and controlled pretreatment process is achieved.

Lignocellulosic materials differ widely in nature, composition, structure etc., and various pretreatment methods also vary considerably. The step of analyzing the lignocellulosic particles of the first slurry to obtain a first data set reflecting the degree of the pretreatment may therefore yield valuable insight towards how to optimize the biomass treatment with the ultimate goal to increase the sugar yield, minimize enzyme consumption and obtaining an energy- and cost-efficient process.

The inventors have found that by analyzing the first slurry and the characteristics of the lignocellulosic particles, the mechanical refining can be adequately controlled; i.e. by adjusting the mechanical refining in response to the first data set.

For instance, the analysis of a pretreated first slurry of a specific lignocellulosic starting material may reveal that the pretreatment was not "efficient enough", and the mechanical refining may then be adjusted to compensate for such insufficient pretreatment in order to achieve sufficient pretreatment and thereby reduce the enzyme consumption in the subsequent enzymatic hydrolysis.

Furthermore, if the same type of pretreatment is used for a different lignocellulosic starting material, the analysis may reveal that only a mild mechanical refining is in fact required. The mechanical refining may then be adapted in response, thereby avoiding unnecessary energy consumptions and saving costs.

The method of the present disclosure thus allows for a more controlled, and situation-adapted pretreatment and hydrolysis which enables swift adjustments in view of the characteristics of the lignocellulosic biomass after pretreatment (the particles of the first slurry). Factors such as degree of pretreatment, composition, chemical and physical nature of the lignocellulosic starting material etc. may therefore be taken into account. Depending on the properties and characteristics of the lignocellulosic particles of the first slurry, the mechanical refining step can be controlled and tailored such that the mechanical refining is adapted to compensate for inadequate pretreatment (when needed) in a sophisticated manner whereby only the necessary energy is used in the process. Such controlled pretreatment in accordance with the present invention allows for savings with respect to the amount of saccharification enzymes to be used during enzymatic hydrolysis (step e). The method of the present disclosure thus allows for large cost savings.

The "lignocellulosic material" may e.g. be selected from hardwoods, softwoods, sugarcane bagasse, sugarcane straw, energy cane, corn stover, corn cobs, corn fibers, straw from rice, wheat, rye and other crops residues.

The term "mechanical refining" refers to a process in which lignocellulosic components or particles are broken down into smaller particles by mechanical means. The mechanical refining may typically involve milling or grinding.

The term "controlling the mechanical refining" means controlling the degree of the mechanical refining. This is accomplished by adjusting the mechanical refining, such as a process parameter of the mechanical refining, in response to the first data set obtained from the analysis of the pretreated material. The step of controlling the mechanical refining may be manual, but is preferably automated and adapted for continuous operation.

The "enzymatic hydrolysis" refers to a process in which at least one saccharification enzyme is added to hydrolyze the pretreated and refined lignocellulosic material into fermentable sugars.

A "saccharification enzyme" refers to an enzyme that can convert or hydrolyze (ligno) cellulosic material into fermentable saccharides, such as monosaccharides and/or disaccharides. Such saccharification enzymes may be glycosidases, which hydrolyze polysaccharides. Examples of glycosidases include cellulose-hydrolyzing glycosidases, such as cellulases, endoglucanases, exoglucanases, cellobiohydrolases and β-glucosidases, hemicellulose hydrolysing glycosidases, such as xylanases, endoxylanases, exoxylanases, β-xylosidases, arabinoxylanases, mannanases, galactanases, pectinases and glucuronases, or any enzymes in the group of enzymes found in EC 3.2.1.x, such as EC 3.2.1.4, where EC is the Enzyme Commission number.

The hydrolysate formed in the process of the present disclosure may comprise sugars from lignocellulosic biomass, such as glucose, mannose, xylose, arabinose, galactose and sucrose.

In embodiments, the method further comprises the step of analyzing the lignocellulosic particles of the second slurry to obtain a second data set reflecting the degree of the mechanical refining of step c).

The step of controlling the mechanical refining may then also be in response to the second data set.

This additional control input improves the performance of the mechanical refining step and enables the subsequent saccharification / hydrolysis to be performed in an efficient manner. Including data from analysis of the second slurry in the control mechanism may serve to ensure that the adequate energy or electrical power is used during the mechanical refining step.

In embodiments, the method further comprises subjecting the second slurry to mechanical refining to form a third slurry comprising lignocellulosic particles, wherein the average size of the lignocellulosic particles of the third slurry is smaller than the average size of the lignocellulosic particles of the second slurry, in response to the second data set.

If the analysis of the lignocellulosic particles of the second slurry reveals that the disintegration was insufficient, the second slurry may be subject to additional mechanical refining, and the third slurry may then be subjected to hydrolysis. Accordingly, the hydrolysis process is improved since a small particle size generally increases the surface area and renders the lignocellulose more accessible to saccharification enzymes. This way, a reduced amount of saccharification enzymes may be used during hydrolysis.

In embodiments, the step c) of controlling the mechanical refining comprises controlling at least one parameter of the mechanical refining; the parameter being selected from:
- the energy input, specific edge load or the electrical power to be used during mechanical refining,
- the residence time in the mechanical refiner, or
- the feed rate or consistency of the first slurry into the mechanical refiner.

By controlling a parameter relating to the mechanical refining in response to a first data set that reflects the degree, or the "severity" of the pretreatment, the subsequent mechanical refining step may be adapted and adjusted to meet the situation-specific requirements, e.g. such that only the necessary amount of energy or electrical power is used. Optimizing and tailoring the mechanical refining process can lead to significant energy saving, lower expenses and less pollution. Furthermore, and importantly, controlling the mechanical refining step allows for a more controlled and efficient pretreatment process. The enzymatic hydrolysis is improved, as a more situation-specific pretreatment process allows for improved quality and stability of the pretreated slurry entering the hydrolysis. This enables to reduce and more efficiently adjust the amount of hydrolytic enzymes used during hydrolysis.

The parameters mentioned above are preferably controlled for the purpose of achieving an efficient mechanical refining. Preferably, the parameters to be controlled are selected from the energy input, specific edge load or the electrical power used during mechanical refining.

"Specific edge load" refers to the refining intensity and may be defined as the amount of energy applied across one meter of a refiner plate's bar edge per second.

These parameters are preferably controlled in response to the first data set of step b). As mentioned, the composition, characteristics and size of the ingoing lignocellulosic material varies greatly, and the ingoing material may therefore respond differently to high vs. low energy intensity. The analysis step thus provides for valuable insight towards how to best optimize the mechanical refining for a specific material and a specific situation.

Furthermore, the mechanical refining and efficiency of the pretreatment largely affects the cellulose-to-sugar conversion yield.

The term "cellulose-to-sugar conversion yield" or "sugar yield", as used herein, means the fraction of sugar monomers and oligomers following hydrolysis relative to the lignocellulose material entering the hydrolysis after the pretreatment and mechanical refining steps.

In embodiments, the step(s) of analyzing the lignocellulosic particles of the first or the second slurry comprises:
- evaluating the amount of sugar and/or sugar degradation biproducts,
- evaluating the proportion between different sugars and/or sugar biproducts,
   - evaluating the amount or presence of pseudo-lignin formed, or
- evaluating the size, shape and/or degree of darkness of the lignocellulosic particles of the first or second slurries.

These parameters reflect the degree of pretreatment, and may be used as means to control the mechanical refining for various types of lignocellulosic materials.

The amount of sugar and/or sugar biodegradation sugar typically involves measuring the total concentration of monomeric sugars. A high concentration of sugar indicates a high severity of the pretreatment, whereas a low concentration indicates a low severity. It should however be noted that a too harsh pretreatment can result in reduced sugar levels due to sugar being degraded and destroyed in the process.

The proportion between different sugars and/or sugar biproducts in the pretreated lignocellulosic composition may also reflect the pretreatment degree and be analyzed to control the mechanical refining. For example, the proportion between glucose and mannose may be used as a measure of how much energy should be used during mechanical refining. The measurement of the concentration of glucose compared to the concentration of mannose is particularly suitable if softwood is used as the lignocellulosic starting material, as disclosed in EP3253892B1, assigned to Sekab E-Technology.

The amount or presence of pseudo lignin also reflects the pretreatment degree. Pseudo-lignin is a lignin like and acid-insoluble material consisting of carbonyl, carboxylic, aromatic and aliphatic structures, which may form a coating on the surface of the polysaccharides. Such pseudo-lignin coating may impair subsequent saccharification by preventing the enzyme access to the carbohydrates of the biomass during enzymatic hydrolysis. A harsh pretreatment may give rise to the undesirable presence of pseudo-lignin, and the mechanical refining step may be used to mechanically break it up and create more available surface for the saccharification enzymes.

Another parameter that reflects the degree of pretreatment and may be used as a parameter to control the subsequent mechanical refining is the size, shape and/or degree of darkness of the lignocellulosic particles.

As mentioned hereinbefore, lignocellulosic particles or components of smaller size are associated with higher enzymatic availability, whereas larger sized components or particles are associated with a lower enzymatic availability. However, the composition of the starting materials may vary greatly, and one selected starting material may not require as much or as severe pretreatment as another. The process conditions and the energy input in the subsequent mechanical refining may thus be adapted to meet the specific situation and the specific starting material.

Another parameter connected to enzymatic availability and improved sugar yield is the degree of darkness of the particles. Typically, darker particles correlate with higher enzymatic availability, whereas brighter particles correlate with lower enzymatic availability. The degree of darkness and the shape of the lignocellulosic particles have previously been demonstrated to correlate to enzymatic availability (in WO2014/114647, assigned to Sekab E-Technology). As demonstrated in WO2014/114647, a "form factor" may be assigned to the lignocellulosic particles. The form factor may be a measure of how spherical or elongated the particles are. By analyzing the shape of the components, the subsequent mechanical refining may be controlled and performed in an optimal manner.

In embodiments, the step(s) of analyzing the lignocellulosic particles of the first slurry and/or the second slurry is an automated online or inline analysis.

As used herein, "inline analysis" refers to evaluation or analysis performed inline; i.e. directly in the process line.

The term "online analysis" refers to evaluation or analysis performed on a sample diverted from the process line, such as in a bypass loop from the main process line.

Automated inline and online analyses are advantageous since time consuming manual sampling methods and subsequent laboratory analyses used for controlling process parameters can be avoided. In other words, inline and online analyses provide a swift and efficient approach for correcting an ongoing process.

In embodiments, the step(s) of analyzing the lignocellulosic particles of the first slurry and/or the second slurry is performed by means of an image analysis method.

An image analysis method is particularly preferred for evaluating the size, shape and/or degree of darkness of the lignocellulosic particles of the first and/or second slurries.

An image analysis method is a direct analysis method which involves extraction of meaningful information from images by means of digital image processing techniques. The image analysis method may comprise taking a digital image e.g. in the visible range and processing the image using an algorithm such that the first data set and/or the second data set is obtained.

The image analysis may be performed by means of an image analysis unit comprising an image capturing device configured to capture images in the visible, infrared (IR) or ultraviolet (UV) spectrum and an image processing device configured to process data received from the image capturing device. Data sets reflecting the degree of pretreatment can thus be obtained.

The image capturing device may be arranged to capture image(s) of the lignocellulosic particles of the first and/or second slurries. The image(s) may e.g. be captured at a position between a pretreatment unit and a mechanical refiner or in a sample diverted from the pretreatment unit. Image(s) may also be captured at a position downstream of the pretreatment unit, e.g. between a mechanical refiner and a hydrolysis unit or in a sample diverted from the mechanical refiner.

The image analysis data may be used to control the mechanical refining and/or for feed-forward control of the enzymatic hydrolysis. For instance, the result of the analysis of the lignocellulosic particles of the second slurry may indicate the amount of enzymes required during enzymatic hydrolysis.

In embodiments, to secure an efficient saccharification and hydrolysis, at least 50%, e.g. at least 70 %, preferably at least 80 % of the lignocellulosic particles of the second slurry has an average diameter in the range of from 0.1 to 500 µm, preferably from 0.1 to 200 µm.

The small size of the particles yields an improved cellulose-to-sugar conversion yield in the subsequent hydrolysis step.

The analysis of the lignocellulosic particles after mechanical refining may serve to secure that the desired size of the particles resulting from pretreatment and mechanical refining has been obtained.

The method of the present disclosure may further comprise the step of controlling at least one process parameter of the pretreatment and/or hydrolysis process in response to the first and/or second data set.

In other words, the analysis of the first and/or second slurry need not only be used for controlling mechanical refining, but other process parameters as well.

The process parameter of the pretreatment and/or hydrolysis process may be selected from:
- a pH-value of the pretreatment step;
- a residence time of the lignocellulosic material in a pretreatment unit or hydrolysis unit of the pretreatment and/or enzymatic hydrolysis step(s);
- a temperature of the pretreatment step;
- a pressure of the pretreatment step; and
- an amount of saccharification enzymes to be added in the enzymatic hydrolysis step.

In other words, the pretreatment and/or hydrolysis process may also be controlled by regulating other parameters, and not only the mechanical refining.

In embodiments, the method further comprises a separation step after the mechanical refining step c).

The separation step serves to remove undesirable liquid from the second slurry. In embodiments, the method according to the present disclosure comprises an additional washing step after the liquid has been removed from the pretreated lignocellulosic composition in step b.

The method of the present disclosure is not limited to a particular pretreatment method, but any type of pretreatment may be used.

In embodiments, the pretreatment comprises steam explosion.

Steam explosion is an energy and cost-efficient pretreatment method for pretreatment of lignocellulosic biomass. During steam explosion, hot steam is used to increase the pressure in the reactor and to disrupt of bonding between polymeric components of the lignocellulosic biomass. Thereafter, decompression from an increased pressure to atmospheric pressure occurs, which results in that the lignocellulose biomass is broken up into smaller particle sizes.

If steam explosion is used as the pretreatment method, the lignocellulosic biomass is disintegrated by size reduction in two steps. The subsequent mechanical refining typically does not need to be as severe if steam explosion is used as the pretreatment method.

According to another aspect, there is provided a system for controlling the pretreatment and/or enzymatic hydrolysis of a lignocellulosic material comprising:
a) a pretreatment unit for pretreating the lignocellulosic material to form a first slurry comprising lignocellulosic particles,
b) means for analyzing the lignocellulosic particles of the first slurry to obtain a first data set reflecting the degree of the pretreatment,
c) a mechanical refiner configured to disintegrate the lignocellulosic particles of the first slurry such that a second slurry comprising lignocellulosic particles having a smaller average size is obtained,
d) an enzymatic hydrolysis unit arranged downstream of and in fluid connection with the mechanical refiner, wherein the system further comprises
e) means for controlling the mechanical refining performed by the mechanical refiner in response to the first data set.

As mentioned hereinbefore, the choice of pretreatment is not limited, and the pretreatment unit may be adapted for any pretreatment method known in the art.

The means for analyzing the lignocellulosic particles may be any means adapted to provide information on the degree or severity of the pretreatment. For example, the means for analyzing the lignocellulosic particles may be based on chemical, spectroscopic or visual analysis.

Alternatively, the analyzing means may be based on image analysis as described hereinbefore. The image analysis means may be an image analysis unit comprising an image capturing device and an image processing device configured to process data received from the image capturing device. The data may be processed by means of software adapted to execute image analysis. The image capturing device may comprise a light source capable of emitting light in the visible spectrum, a camera lens and a digital camera capable of capturing an image in the visible spectrum.

The enzymatic hydrolysis unit is configured to hydrolyze the second slurry by means of saccharification enzymes.

The means for controlling at least one parameter of the mechanical refining may be automated inline or online control means, such as computerized control means.

In embodiments, the means for analyzing the lignocellulosic particles of the first slurry comprises an image analysis unit configured to provide information about the size, shape and/or degree of darkness of the lignocellulosic particles.

According to another aspect, there is provided a system for controlling the treatment of lignocellulosic material comprising a system as described hereinbefore and a fermentation unit arranged in fluid communication with and downstream of the system.

Further features of, and advantages with, the present disclosure will become apparent when studying the appended claims and the following description. The skilled addressee realizes that different features of the present disclosure may be combined to create embodiments other than those described in the following, without departing from the scope of the present disclosure.

Terms, definitions and embodiments of the first aspect of the present disclosure apply mutatis mutandis to the other aspects of the present disclosure, and vice versa.

### Brief description of the drawings

The various aspects of the present disclosure, including its particular features and advantages, will be readily understood from the following detailed description and the accompanying drawings, in which
Figure 1 schematically illustrates the steps of a method for controlling the pretreatment and/or enzymatic hydrolysis according to the present disclosure.
Figure 2 schematically illustrates a system for controlling the pretreatment and/or enzymatic hydrolysis according to an exemplary embodiment of the present disclosure.

### Detailed description

The present invention will now be described more fully hereinafter with reference to the accompanying drawings. The present invention may, however, be embodied in many different forms and should not be construed as limited to the embodiments set forth herein; rather, these embodiments are provided for thoroughness and completeness, and fully convey the scope of the present invention to the skilled person.

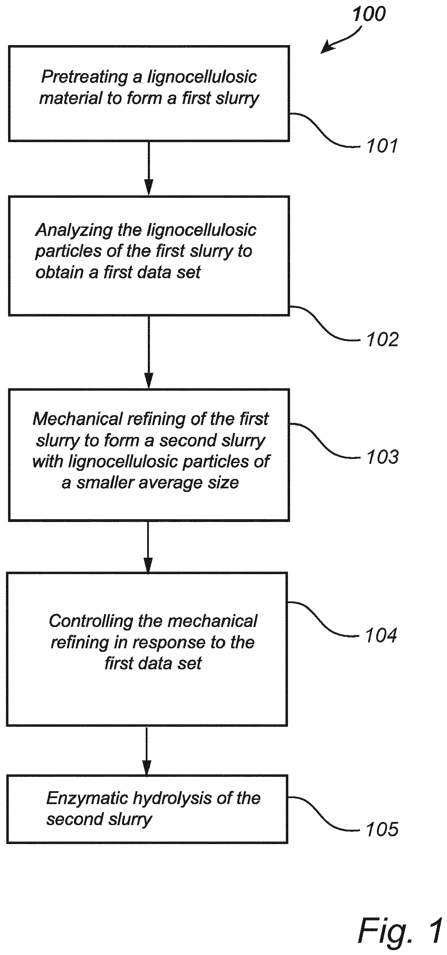

Figure 1 schematically illustrates the steps of a method (100) for controlling the pretreatment and/or enzymatic hydrolysis, according to the present disclosure.

First, a lignocellulosic material is pretreated to form a first slurry comprising lignocellulosic particles (101).

Next, the lignocellulosic particles of the first slurry are analyzed to obtain a first data set reflecting the degree of the pretreatment (102).

The first slurry is subjected to mechanical refining to form a second slurry comprising lignocellulosic particles, wherein the average size of the lignocellulosic particles of the second slurry is smaller than the average size of the lignocellulosic particles of the first slurry (103). The mechanical refining may e.g. be accomplished by milling or grinding.

The method (100) further comprises the step of controlling the mechanical refining in response to the first data set (104). The purpose of this step is to adapt and adjust the mechanical refining in response to the first data set such that sufficient mechanical refining is achieved for the specific lignocellulosic material to be pretreated. Furthermore, the control step serves to secure that energy is not consumed superfluously.

The second slurry is thereafter subjected to enzymatic hydrolysis (105). A hydrolysate is thereby obtained, which may be fermented and converted to a target chemical in downstream processes.

The method may further comprise at least one separation step to remove pretreatment liquid from the first and/or second slurries.

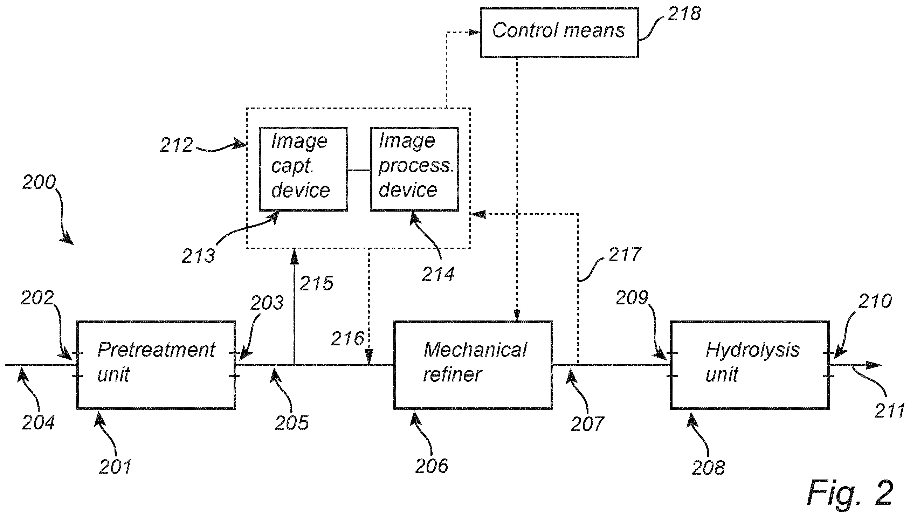

Figure 2 schematically illustrates a system 200 system for controlling the pretreatment and/or enzymatic hydrolysis of a lignocellulosic material. The system 200 comprises a pretreatment unit 201 for pretreating the lignocellulosic material to form a first slurry 205 comprising lignocellulosic particles. The pretreatment unit 201 may e.g. comprise a reactor, a vessel or a container. The pretreatment unit 201 comprises an inlet 202 and an outlet 203. A feed stream of a lignocellulosic material 204 enters the pretreatment unit 201 through the inlet 202, and a first slurry 205 comprising lignocellulosic particles is discharged through the outlet 203. The first slurry 205 comprises a liquid component and a solid component (comprising lignin and cellulose and/or hemicellulose). At least part of the liquid component may, if desired, be removed from the stream 205 in a separation unit (not shown). Such a separation unit may e.g. comprise a screen, a filter, a decanter screw, a centrifuge or similar equipment to separate and remove the liquid component from the first slurry 205.

The system 200 further comprises a mechanical refiner 206 configured to disintegrate the lignocellulosic particles of the first slurry 205 such that a second slurry 207 comprising lignocellulosic particles having a smaller average size is obtained. The mechanical refiner 206 may be any suitable type of mill or a single or double disc refiner. Preferably, the mechanical refiner 206 is a disc refiner.

The mechanical refiner may comprise disc segments configured to disintegrate and/or defibrate the particles of the first slurry 205. Preferably, the mechanical refiner 206 is configured to disintegrate the lignocellulosic particles such that at least 50%, e.g. at least 70%, preferably at least 80% of the lignocellulosic particles have a diameter in the range of from 0.1 to 500 µm, e.g. from 0.1 to 200 µm. The mechanical refiner 206 may also comprise means for heating and/or pressurizing the lignocellulosic composition.

The system 200 further comprises an enzymatic hydrolysis unit 208 arranged downstream of and in fluid connection with the mechanical refiner 206. In the hydrolysis unit 208, the pretreated biomass (i.e. the second slurry) is subject to enzymatic hydrolysis by means of saccharification enzymes. The hydrolysis unit 208 comprises an inlet 209 for receiving the second slurry 207, and an outlet 210 for discharging the hydrolysate 211 formed after enzymatic hydrolysis.

The hydrolysate 211 may be then be subjected to separation to remove residual solids formed during hydrolysis. The hydrolysis unit 208 may be arranged in fluid communication with a fermentation vessel (not shown). Fermentation of the hydrolysate into a target chemical is typically performed by means of fermenting organism, such as bacteria and/or yeast. The system 200 may also comprise a product recovery unit, such as distillation or ion exchange chromatography, arranged downstream of and in fluid communication with the fermentation vessel.

The system further comprises means for analyzing the lignocellulosic particles of the first slurry 205 to obtain a first data set reflecting the degree of the pretreatment. In figure 2, the means for analyzing is an image analysis unit 212 comprising an image capturing device 213 and an image processing device 214. The image capturing device 213 may comprise a light source capable of emitting light in the visible spectrum, a camera lens and a digital camera capable of capturing an image in the visible spectrum. The image processing device 214 is connected to the image capturing device 213 and is capable of processing data received from the image capturing device 213, e.g. by means of software adapted to execute image analysis. A data set that reflects the degree of pretreatment is thus obtained, and this data set can be used to control the mechanical refining. For example, in the case of image analysis, information about the size, darkness or form of the lignocellulosic particles can be obtained. A sample 215 of the first slurry 205 may be diverted from the process line and subject to image analysis prior to mechanical refining. The analyzed and/or excess sample 215 can either be discarded or returned to the enzymatic hydrolysis process through a sample return inlet 216. A sample 217 of the second slurry 207 may also be diverted from the process line and subject to analysis.

The system 200 further comprises means 218 for controlling the mechanical refining of the mechanical refiner 206 in response to the first data set, so as to disintegrate the particles of the slurry further depending on how well the primary pretreatment has processed the particles. The control means 218 is preferably an automated control means, such as computer control means, suitable for online or inline control.

The means 218 for controlling the mechanical refining is arranged to communicate with the image processing device 214 of the image analysis unit 212 and with the mechanical refiner 206. The control means 218 typically receives signals, e.g. data reflecting the degree of pretreatment, from the image analysis unit 212 and outputs signals to the mechanical refiner 206 that controls the mechanical refining of the system according to predetermined control criteria. This may in embodiments involve setting the energy input, specific edge load, or the electrical power of the mechanical refiner 206, so as to optimize the overall pretreatment. Minimal energy input (basically corresponding to no refining) may be used when the data reflecting the degree of pretreatment shows that no further disintegration in the refining step is needed. The skilled person understands that the actual control algorithms and signal handling can involve conventional control means or regulators.

The means 218 for controlling the mechanical refining may in embodiments be arranged in the same physical processing unit as the image processing device 214.

Even though the present disclosure has been described with reference to specific exemplifying embodiments thereof, many different alterations, modifications and the like will become apparent for those skilled in the art.

Variations to the disclosed embodiments can be understood and effected by the skilled addressee in practicing the present disclosure, from a study of the drawings, the disclosure, and the appended claims. Furthermore, in the claims, the word "comprising" does not exclude other elements or steps, and the indefinite article "a" or "an" does not exclude a plurality.

## Claims

1. A method for controlling the pretreatment and/or enzymatic hydrolysis of a lignocellulosic material comprising:
a) pretreating a lignocellulosic material to form a first slurry comprising lignocellulosic particles
b) analyzing the lignocellulosic particles of said first slurry to obtain a first data set reflecting the degree of the pretreatment,
c) subjecting said first slurry to mechanical refining to form a second slurry comprising lignocellulosic particles, wherein the average size of the lignocellulosic particles of said second slurry is smaller than the average size of the lignocellulosic particles of said first slurry,
d) controlling said mechanical refining in response to said first data set; and
e) subjecting said second slurry to enzymatic hydrolysis.

2. The method according to claim 1, wherein said method further comprises the step of analyzing the lignocellulosic particles of said second slurry to obtain a second data set reflecting the degree of said mechanical refining of said step c).

3. The method according to claim 2, wherein said step d) of controlling said mechanical refining is also in response to said second data set.

4. The method according to claim 2 or claim 3, further comprising subjecting said second slurry to a second step of mechanical refining to form a third slurry comprising lignocellulosic particles, wherein the average size of the lignocellulosic particles of said third slurry is smaller than the average size of the lignocellulosic particles of said second slurry, in response to said second data set.

5. The method according to any one of the preceding claims, wherein said step d) of controlling said mechanical refining comprises controlling at least one parameter of said mechanical refining; said parameter being selected from:
- the energy input, specific edge load or the electrical power to be used during mechanical refining,
- the residence time in the mechanical refiner, or
- the feed rate or consistency of said first slurry into the mechanical refiner.

6. The method according to any one of the preceding claims, wherein the step(s) of analyzing the lignocellulosic particles of said first and/or said second slurry comprises:
- evaluating the amount of sugar and/or sugar degradation biproducts,
- evaluating the proportion between different sugars and/or sugar biproducts,
- evaluating the amount of pseudo-lignin formed
- evaluating the size, shape and/or degree of darkness of the lignocellulosic particles of said first and/or second slurries.

7. A method according to any one of the preceding claims, wherein said step(s) of analyzing said lignocellulosic particles of said first slurry and/or said second slurry is an automated online or inline analysis.

8. A method according to any one of the preceding claims, wherein the step(s) of analyzing the lignocellulosic particles of said first slurry and/or said second slurry is performed by means of an image analysis method.

9. The method of any one of the preceding claims, wherein at least 50 %, e.g. at least 70 %, preferably at least 80 % of the lignocellulosic particles of said second slurry has an average diameter in the range of from 0.1 to 500 µm, preferably from 0.1 to 200 µm.

10. The method according to any one of the preceding claims, further comprising the step of controlling at least one process parameter of the pretreatment and/or hydrolysis process in response to said first and/or second data set.

11. The method of claim 10, wherein said process parameter of the pretreatment and/or hydrolysis process is selected from:
- a pH-value of the pretreatment step;
- a residence time of the lignocellulosic material in a pretreatment unit or hydrolysis unit of the pretreatment and/or enzymatic hydrolysis step(s);
- a temperature of the pretreatment step;
- a pressure of the pretreatment step; and
- an amount of saccharification enzymes to be added in the enzymatic hydrolysis step.

12. The method of any one of the preceding claims, wherein said method further comprises a separation step after said mechanical refining step c).

13. The method of any one of the preceding claims, wherein said step a) of pretreating said lignocellulosic material comprises steam explosion.

14. A system (200) for controlling the pretreatment and/or enzymatic hydrolysis of a lignocellulosic material comprising:
a) a pretreatment unit (201) for pretreating the lignocellulosic material to form a first slurry comprising lignocellulosic particles,
b) means (212) for analyzing the lignocellulosic particles of the first slurry to obtain a first data set reflecting the degree of the pretreatment,
c) a mechanical refiner (206) configured to disintegrate the lignocellulosic particles of the first slurry such that a second slurry comprising lignocellulosic particles having a smaller average size is obtained,
d) an enzymatic hydrolysis unit (208) arranged downstream of and in fluid connection with said mechanical refiner (206), wherein the system (200) further comprises
e) means for controlling the mechanical refining performed by said mechanical refiner (206) in response to said first data set.

15. The system (200) of claim 14, wherein said means (212) for analyzing the lignocellulosic particles of said first slurry comprises an image analysis unit configured to provide information about the size, shape and/or degree of darkness of the lignocellulosic particles.

16. A system for controlling the treatment of lignocellulosic material comprising the system (200) for controlling the pretreatment and/or hydrolysis of a lignocellulosic material according to claim 14 or claim 15 and a fermentation unit arranged in fluid communication with and downstream of said system (200).
